# EUROPEAN PATENT SPECIFICATION

(11) **EP 3 170 477 B1**
(45) Date of publication and mention of the grant of the patent: **19.02.2020**
(21) Application number: 16198305.1
(22) Date of filing: 11.11.2016
(51) Int. Cl.: A61F 2/30, A61B 17/04, A61F 2/42

(54) **SCAPHOID PROSTHESIS**
KAHNBEINPROTHESE
UNE PROTHÈSE POUR LE SCAPHOÏDE

(30) Priority: 22.11.2015 EP 15195745
(43) Date of publication of application: 24.05.2017
(73) Proprietor: Medartis Holding AG, 4057 Basel (CH)
(72) Inventor: Honigmann, Dr Philipp, 4410 Liestal (CH); Häfeli, Dr Mathias, 5607 Hägglingen (CH); Thieringer, DDr Florian, 4056 Basel (CH); Schumacher, Dipl. Ing. Ralf, 4411 Seltisberg (CH)
(74) Representative: Müller, Christoph Emanuel

(56) References cited:
- WO-A1-2009/076758
- US-A- 5 702 468
- US-B1- 6 371 985
- GARCIA-ELIAS ET AL: "Three-Ligament Tenodesis for the Treatment of Scapholunate Dissociation: Indications and Surgical Technique", THE JOURNAL OF HAND SURGERY, W.B. SAUNDERS, AMSTERDAM, NL, vol. 31, no. 1, 1 January 2006 (2006-01-01), pages 125-134, XP005266142, ISSN: 0363-5023, DOI: 10.1016/J.JHSA.2005.10.011
- CORELLA FERNANDO ET AL: "Arthroscopic Ligamentoplasty of the Dorsal and Volar Portions of the Scapholunate Ligament", THE JOURNAL OF HAND SURGERY, vol. 38, no. 12, 31 December 2013 (2013-12-31), pages 2466-2477, XP028783326, ISSN: 0363-5023, DOI: 10.1016/J.JHSA.2013.09.021

## Description

The invention is related to a scaphoid prosthesis. The scaphoid is the most important carpal bone. Because of the distally based blood supply, healing of fractures is at risk because the proximal pole has no blood supply and therefore only bad healing potential. If a fracture does not heal, a pseudoarthrosis will develop. Untreated, the pseudoarthrosis will lead to destruction of the joint cartilage (arthrosis) and inflammation (arthritis) with pain, loss of range of motion and function.

A number of conventional approaches are available depending on the severeness of the pseudoarthrosis developed in consequence of an undetected and consequently untreated or unhealed fracture. The treatments range from placing a non-vascularized or vascularized bone graft to reconstitute the patient's scaphoid to ultimately a fusion of the carpal bones or to a resection of the first carpal row in a proximal row carpectomy.

Already in 1945, a patient specific prosthetic replacement of the scaphoid was developed using Vitallium (cobalt, chrome and molybdenum alloy). Very little is known about the use and results in the literature. Agner developed an Acrylate prosthesis in 1954 and used this prosthesis in patients [1]. Severe complications like foreign body reaction to silicone with the development of granulomas were reported. In addition, the carpal collapse could not be prevented. Although these problems were well known, Swanson brought another silicone prosthesis 1962 on the market with the same complications, such as the one disclosed e.g. in US 4 164 793A or US 4 158 893 A.

In 1989 Swanson reacted on these complications and developed a non-anatomical prosthesis made of titanium, such as the one disclosed in US 4 645 505 A. There is no information upon the use of this kind of prosthesis in the literature.

Another type of placeholder is the prosthesis made of pyrocarbon by Tornier named Amandys also without any functional or biomechanical suspension or attachment to the carpal bones. An example for a composite prosthesis made of pyrocarbon and metal has been disclosed e.g. in WO2008001185 A2. The only functional and biomechanical attached prosthesis for the carpus is an implant for the lunate made of pyrocarbon by Ascension, as disclosed in US2005033426A1.

A publication in 2011 reported on a custom-made prosthesis made by titanium by Spingardi/Rossello [2] who reported on the implantation in 113 patients. Five of them dislocated within the follow-up period of 12 years.

All these prostheses have the same problems, in that they are not biomechanical compatible and just act as a spacer without any suspension or link to the carpal arrangement. These spacers have a major complication: they tend to luxate and cannot prevent carpal collapse.

From US6371985B1 it is known to fix prostheses to bones whereby channels are drilled in these prostheses. It is intended that the bone grows into these channels. Neither the tendon nor the prosthesis can move/glide anymore. Such a prosthesis would not meet the biomechanical need and consequently prevent the patient from regaining most of the flexibility of the hand, therefore the application of this technique for hand surgery appears to be unsuitable.

According to US 5 702 468 A1, a surgically implantable carpal bone prosthesis is provided, which comprises a biocompatible, medically inert body member contoured to resemble the shape of the carpal bone, which it is to replace. The body member contains two independent channels, which are used for means for restraining the body member along crisscrossing axes. The constrained prosthesis is fixed by drilling a channel through the lunate where the tendon in the technique of Henry / Corella is passed through and sutured to itself to biomechanically reconstruct the dorsal and palmar scapho-lunate ligaments to ensure physiological movement of the prosthesis.

It is also known from WO2009076758A1 to produce an anatomical replica of a scaphoid bone based on images of the scaphoid bone of the contralateral wrist, i.e. a mirror image using computer tomography or magnetic resonance scans.

The objective is thus to develop a patient-specific prosthesis for the scaphoid bone of increased strength and stability which shall replace the pseudoarthrotic/non-reconstructable scaphoid in cases of impossible or failed attempts of reconstruction.

The problem is thus solved by the subject matter as claimed in claim 1. Further advantageous embodiments are subject to the dependent claims 2 to 12. The scaphoid prosthesis according to the subject matter of any of claims 1 to 12 can advantageously be manufactured by any of the methods forming the subject matter of claims 13 to 15.

In other words, the problem is solved by providing a more accurate scaphoid prosthesis matching with the patient's scaphoid which is suitable for interaction with the portions of the anatomic structure not affected by pseudoarthrosis. For obtaining a more accurate scaphoid prosthesis, a modelling of the patient's scaphoid has been performed to provide a more accurately shaped scaphoid prosthesis.

The scaphoid prosthesis comprises a body bounded by an outer surface, whereby the outer surface is substantially corresponding to a patient's scaphoid. The body of the scaphoid prosthesis comprises a tubular base element including a first end portion and a second end portion and a plurality of protruding portions. A single curved passage is provided in the tubular base element for a fixation means for fixing the scaphoid prosthesis in its position. The curved passage is positioned in the body in such a way that the distance between the passage wall and the body surface is substantially uniform, that means the passage is arranged in a central region of the body. In particular, the distance between the passage wall and the outer surface measured along any line intersecting with the longitudinal axis is substantially uniform in any cross-sectional area arranged normally to the longitudinal axis of the passage. The advantage of adapting the curvature of the passage to the surface structure of the outer surface of the body is to maximize the body volume surrounding the passage in almost any position of the passage.

A scaphoid prosthesis is thus generated from a scaphoid model, whereby the scaphoid model is generated from patient data and it corresponds in its shape to the patient's scaphoid.

Under a scaphoid model, it is to be understood a computer generated three-dimensional image of the patient's scaphoid. An image of the patient's scaphoid can be obtained by state-of-the art imaging technologies, such as X-ray imaging or MRI imaging, which can be available in a database. Due to the fact, that the shape of the scaphoid prosthesis is known from the scaphoid model, it is possible to calculate the curvature of the passage from the shape as given by the scaphoid model. Thereby the body can be anatomically contoured based on data of the contralateral side or from the database. The boundary condition for obtaining the optimum curvature is determined by setting the distance between the passage wall and the outer surface to be substantially uniform, thus to be substantially the same. Thus, the passage is arranged in the scaphoid model in such a manner, that the distance from the passage wall to the outer surface is substantially the same for any cross-sectional area arranged normally to the longitudinal axis of the passage.

There is a need to provide a patient specific prosthesis mounted in an anatomical structure, such as a bone assembly of a wrist. In particular, if treatment of a joint is required, it is required that the position of a plurality of engaging or interacting anatomical structures is aligned.

A scaphoid prosthesis comprises a body bounded by an outer surface, whereby the outer surface is substantially corresponding to a patient's scaphoid. The body of the scaphoid prosthesis comprises a tubular base element including a first end portion and a second end portion and a plurality of protruding portions.

A partial arthrodesis (4-corner fusion) or removal of the first carpal row by proximal row carpectomy (PRC) can be avoided using the scaphoid prosthesis according to the invention. In particular, by using a scaphoid prosthesis according to the invention, the anatomy and biomechanics of the wrist can be maintained. If the prosthetic replacement of the scaphoid should fail, the application of the prior art techniques remains possible.

To meet the biomechanical requirement of the wrist the prosthesis is functionally suspended using the surgical technique mentioned below. This functional and biomechanical aspect is a unique feature of the implant. The biomechanical and functional suspension of the prosthesis derives from known procedures for scapholunate ligament reconstruction, which is the ligament between the scaphoid and the lunate bone and one of the main stabilizers of the carpus. Other ligaments for the stabilization of the carpal bone are so called secondary stabilizers and are ligaments between the scaphoid and the carpus other than the scapholunate ligament. The technique for the scapholunate ligament reconstruction is performed to anchor the scaphoid prosthesis in the biomechanically correct position. Thereby, a good fixation of the prosthesis is obtained and in addition, luxation and carpal collapse are prevented, which would finally lead into the development of carpal arthrosis.

According to the invention, a single curved passage is provided in the tubular base element for a fixation means for fixing the scaphoid prosthesis in its position. The fixation is advantageously obtained by a tendon strip of the Flexor Carpi Radialis tendon (FCR) passing through the passage in the scaphoid prosthesis. In particular, the tubular base element can have a longitudinal axis substantially corresponding to the opening in the body of the scaphoid prosthesis. The longitudinal axis can extend substantially from the first end portion to the second end portion. The passage can comprise an attachment portion, which can be formed in particular as one of a threaded portion or a roughened portion. The attachment portion can have a smaller cross-sectional area than at least one of the ends of the passage. The threaded portion may be used for fixing a fixation element.

According to an embodiment, the passage is composed of a first hole and a second hole, whereby the first hole comprises a first longitudinal axis and the second hole comprises a second longitudinal axis. The first and second longitudinal axes are arranged in an angle to each other. One of the first or second holes is advantageously disposed with an attachment portion. According to an embodiment, the surface of the passage can include a roughened portion or a threaded portion. The attachment portion may form an anchoring portion for an interference screw. An interference screw can be used to fix the ligament in the scaphoid prosthesis and/or the lunate to increase stability.

According to an embodiment, the scaphoid prosthesis can comprise protruding portions having a roughly spherical or ellipsoid shape twisted about the longitudinal axis. In particular, the twisting angle of the first end portion relative to the second end portion can be about 90 degrees.

According to an embodiment, the scaphoid prosthesis comprises a scaphoid model, wherein the scaphoid model is obtainable from patient data and corresponds in its shape substantially with the patient's scaphoid, whereby the scaphoid prosthesis is obtained from the scaphoid model. In other words, the scaphoid prosthesis is created according to this embodiment utilizing a scaphoid model, wherein the scaphoid model is generated utilizing patient scaphoid data, and wherein the shape of the scaphoid model represents the shape of the scaphoid prosthesis. The scaphoid model can represent a replacement scaphoid, such that the shape of the replacement scaphoid has an outer surface forming the surface of the scaphoid prosthesis, which has substantially the same shape as the surface of the patient's scaphoid. In particular, the scaphoid model is designed by a computer aided design software using the patient data for calculating a shape of a replacement scaphoid of the shape of the patient's scaphoid. The shape of the replacement scaphoid can have an outer surface forming the surface of the scaphoid prosthesis, which has substantially the same shape as the surface of the patient's scaphoid. Thereby a patient specific scaphoid prosthesis is obtainable.

The scaphoid prosthesis according to any of the preceding embodiments is made from a biocompatible material suitable for permanent reception in a human body. Preferably, the scaphoid prosthesis is made from a biocompatible material. The material can comprise at least one element from the group consisting of titanium, a biocompatible plastic or a polymer, such as a polyetheretherketone or a ceramic material, for instance a ceramic material containing zirconia.

According to an embodiment, an opening is provided in the scaphoid prosthesis for a fixation means for fixing the scaphoid prosthesis in its position.

The scaphoid prosthesis can comprise a supporting structure extending between the passage and the body surface. The supporting structure can comprise at least one element from the group grids, webs, porous structures, fibers. The supporting structure can be filled by a filler material. The supporting structure can provide the required mechanical stability, whereas the filler material can comprise any biocompatible material such as the materials previously mentioned. The body surface may be formed by a skin, such that the supporting structure is shielded from the environment.

The scaphoid prosthesis according to any of the preceding embodiments can be obtainable by an additive manufacturing method. In particular, a method for manufacturing a scaphoid prosthesis can comprise an additive manufacturing step. Furthermore, the method for manufacturing a scaphoid prosthesis can comprise a first step to obtain data relating to the shape of a patient's scaphoid, in a second step a scaphoid model is generated by a computer aided design software, wherein the scaphoid model which is generated from patient data, corresponds in its shape substantially with the patient's scaphoid, whereby the scaphoid prosthesis can be obtained from the scaphoid model in a third step by the additive manufacturing method.

A method for manufacturing a scaphoid prosthesis according to any of the previously mentioned embodiments can comprise a shaping or forming process starting from a raw material or an intermediate product. In particular, the scaphoid prosthesis is formed from a tubular element comprising a plurality of ribbon-shaped elements, whereby the outer shape of the scaphoid prosthesis is shaped by moving the first end portion towards the second end portion such that a roughly spherical or ellipsoidal shape is obtained, whereby the first end portion is twisted relative to the second end portion such that a scaphoid shape is obtained which corresponds roughly to the surface of the patient's scaphoid.

An aspect of the disclosure relates to a method for manufacturing a scaphoid prosthesis comprising a body bounded by an outer surface, wherein the outer surface is substantially corresponding to a patient's scaphoid, wherein the body of the scaphoid prosthesis comprises a tubular base element including a first end portion and a second end portion and a plurality of protruding portions wherein a single passage is provided in the tubular base element configured to engage a fixation means for fixing the scaphoid prosthesis in its position, wherein the passage is configured as a curved passage, the method comprising an additive manufacturing step.

In one embodiment, the method comprising, by a computing device, receiving data relating to the shape of a patient's scaphoid and generating a scaphoid model, wherein the shape of the scaphoid model corresponds to the shape of the patient's scaphoid.

In one embodiment, the method comprising, by a computing device, generating a scaphoid model utilizing data relating to the shape of a patient's scaphoid.

The invention will be explained in more detail in the following with reference to the drawings obtained from [3]/[4]. There are shown in a schematic representation in:
Fig. 1a view on the carpal bones,
Fig. 2 the blood supply of the scaphoid,
Fig. 3a an x-ray scan of a pseudoarthrosis of the scaphoid,
Fig. 3b a MRI scan of a pseudoarthrosis of the scaphoid,
Fig. 4a the first stage of SNAC,
Fig. 4b the second stage of SNAC,
Fig. 4c the third stage of SNAC,
Fig. 5a a prior art treatment of pseudoarthrosis of the scaphoid involving the integration of a non-vascularized bone graft into the patient's scaphoid,
Fig. 5b the second stage of the treatment according to Fig. 5a,
Fig. 5c the third stage of the treatment according to Fig. 5a,
Fig. 5d the fourth stage of the treatment according to Fig. 5a,
Fig. 5e a prior art treatment of pseudoarthrosis of the scaphoid involving the integration of a local vascularized bone graft from the dorsal side of the distal radius into the patient's scaphoid,
Fig. 5f a second stage of the treatment according to Fig. 5,
Fig. 5g variant of the treatment according to Fig. 5a or Fig. 5b using a local vascularized bone graft from the palmar side of the distal radius,
Fig. 5h a prior art treatment of pseudoarthrosis of the scaphoid involving the integration of a free vascularized bone graft from the knee into the patient's scaphoid,
Fig. 6a a prior art treatment of pseudoarthrosis of the scaphoid involving a resection of the scaphoid,
Fig. 6b the prior art treatment of pseudoarthrosis of the scaphoid according to Fig. 6a involving a partial fusion of the carpal bones also referred to as a 4-corner fusion,
Fig. 6c and x-ray scanned image of a treatment according to Fig. 6a or Fig. 6b,
Fig. 7 a view on the wrist bones after a proximal row carpectomy,
Fig. 8a a first view of a scaphoid prosthesis according to a first embodiment of the invention,
Fig. 8b a second view of the scaphoid prosthesis according to Fig. 8a,
Fig. 8c a first view of a scaphoid prosthesis according to a second embodiment of the invention,
Fig. 9a-9c a technique for attaching a scaphoid prosthesis to a carpal bone structure according to a first variant,
Fig. 9d a technique for attaching a scaphoid prosthesis to a carpal bone structure according to a second variant,
Fig. 9e a section of a scapholunate ligament,
Fig. 10 a technique for attaching a scaphoid prosthesis to a carpal bone structure according to a third variant,
Fig. 11a-j a series of steps of a technique for attaching a scaphoid prosthesis to a carpal bone structure according to a fourth variant,
Fig. 12 a block diagram of a computing device.

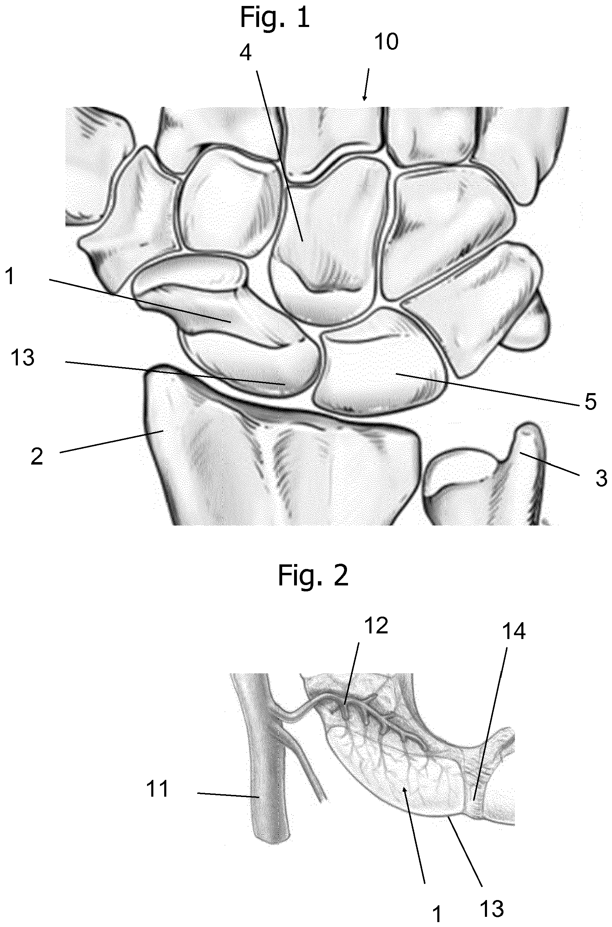

Fig. 1 shows the position of the scaphoid 1 in a human wrist 10. Fig. 1 shows thus the bones of a left hand in a dorsal view. The scaphoid 1 is the most important carpal bone connecting the radius 2 and the ulna 3 with the capitate 4 and the lunate 5.

Fig. 2 shows a detail of the blood supply to the scaphoid 1 as depicted in Fig. 1. The sectional view of Fig. 2 shows a portion of a main blood vessel 11 and a branching blood vessel 12 alimenting the scaphoid 1. Because of the distally based blood supply, healing of fractures is at risk because the proximal pole 13 has no blood supply and therefore only bad healing potential. The scaphoid is connected to the lunate with the scapho-lunate ligament 14. If a fracture of the scaphoid 1 does not heal, a pseudoarthrosis will develop. If untreated, the pseudoarthrosis will lead to destruction of the joint cartilage and an arthrosis develops together with an inflammation or arthritis resulting in pain, loss of range of motion and function.

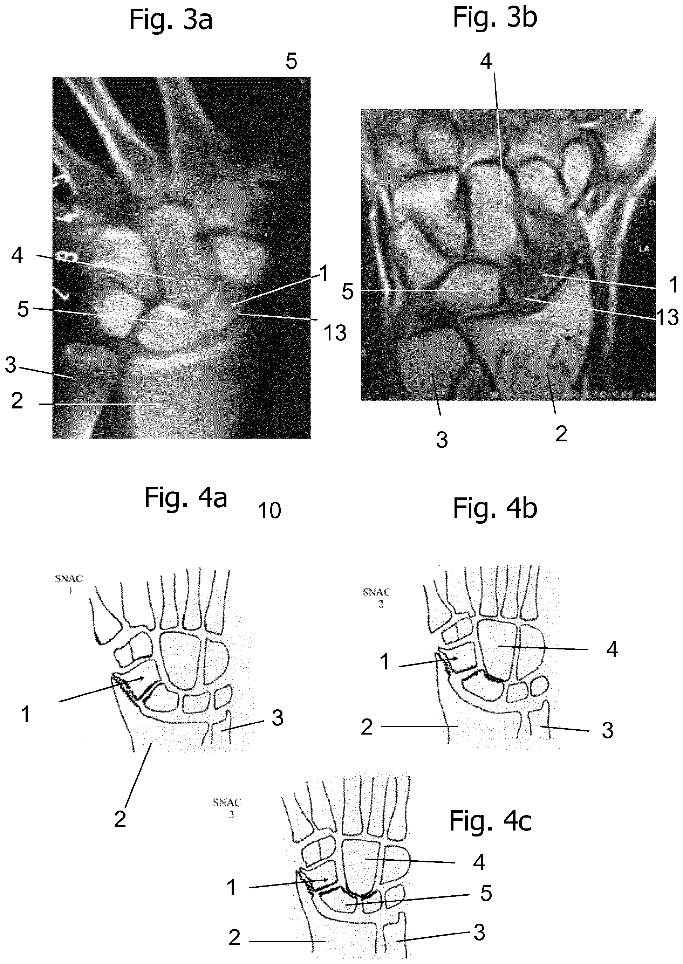

Figure 3a shows a pseudoarthrosis of the scaphoid 1 on a left hand in an image, which was obtained by conventional x-ray. The MRI-scan in Fig. 3b shows a decreased perfusion of the proximal pole 13. The area of decreased perfusion is shown as a dark colored Scaphoid bone compared to the other brighter carpal bones, e.g. the neighboring capitate 4 or lunate 5.

The development of the arthrosis follows a defined process and results finally in a collapse of the biomechanical important carpal alignment, which will finally lead to a complete arthrosis of the wrist. Figure 4a - c show the stages of development of the arthrosis in case of scaphoid nonunion 1. In the first stage of the so-called "Scaphoid Nonunion Advanced Collapse" (= SNAC) arthrosis is shown in Fig. 4a and develops between the styloid of the radius 2 and the scaphoid 1 bone. The second stage, as shown in Fig. 4b, additionally affects the midcarpal joint between capitate 4 and the scaphoid 1 where arthrosis develops. During the third stage, as shown in Fig. 4c, the midcarpal joint between scaphoid and lunate 5 bone is also affected. Surgical treatment of the scaphoid pseudoarthrosis according to the prior art consists of a resection of the pseudoarthrosis and reconstruction of the scaphoid using a non-vascularized bone graft (i.e. from the iliac crest).

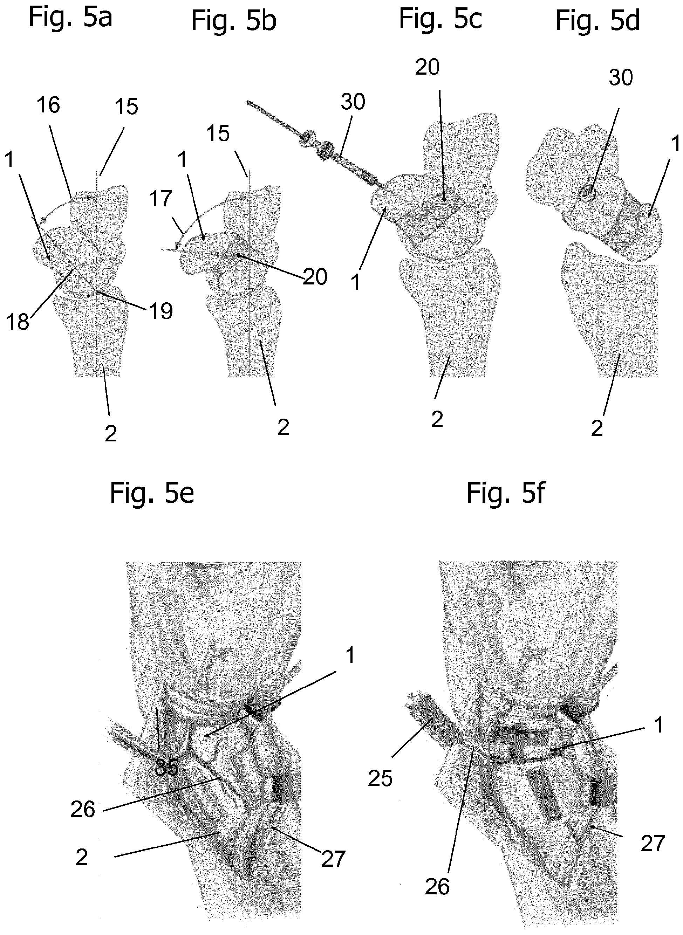

Fig. 5a shows the normal positioned scaphoid. Angle 16 is the scapho-lunate angle (S-L angle) which is calculated between an orthogonal line 15 through the lunate and a line, which lies exactly in the axis 18 of the scaphoid 1. Normal values range from 30 to 70 degrees.

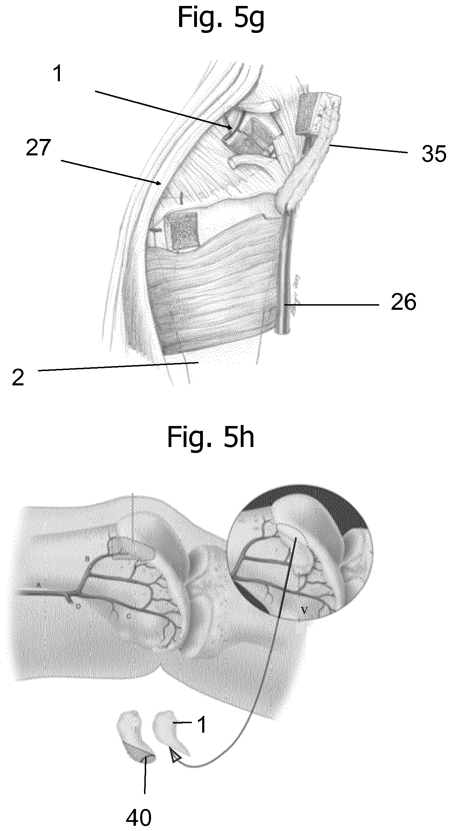

Fig. 5b shows a scaphoid in a mal-united position. The scaphoid has like a "humpback" why this mal-united position is called a humpback-deformity. Angle 17 has a value greater than 70 degrees. A correct placed bone graft 20 leads to the results seen in Fig. 5c where the scaphoid 1 is in anatomical position and the S-L-angle 16 is normal. This bone graft 20 is then fixed using a compression screw 30 seen in Fig. 5d. In cases of a vascularity of the proximal pole, a local vascularized bone graft 25 is used (fig. 5e, fig. 5f or fig. 5g). Fig. 5e shows the dorsal portion of the distal radius 2 containing a blood supply 26 after removal of the tissue layers 27 as suggested by Zaidemberg [5]. The treatment of pseudoarthrosis of the scaphoid 1 involves the integration of a local vascularized bone graft 20 from the dorsal side of the distal radius 2 into the patient's scaphoid 1. Fig. 5g shows a variant of a local vascularized bone graft 35 from the palmar side of the distal radius 2 as suggested by Kuhlmann/Mathoulin [6].

Alternatively a free vascularized bone graft 40 can be used taken from another location in the body, such as e.g. from the medial femoral condyle as shown in Fig. 5h as suggested by Bürger [7]. Fig. 5h also shows a model of the scaphoid 1 from which the avascular proximal segment has been removed by resection. The resected segment from the knee is attached to the scaphoid 1 shown in the model depicted on the left side of the resected scaphoid 1. A possible origin of the free vascularized bone graft 40 is shown by the curved arrow pointing to the location on the medial femoral condyle from which the osteo-cartilaginous graft is harvested to recreate the scaphoid proximal pole.

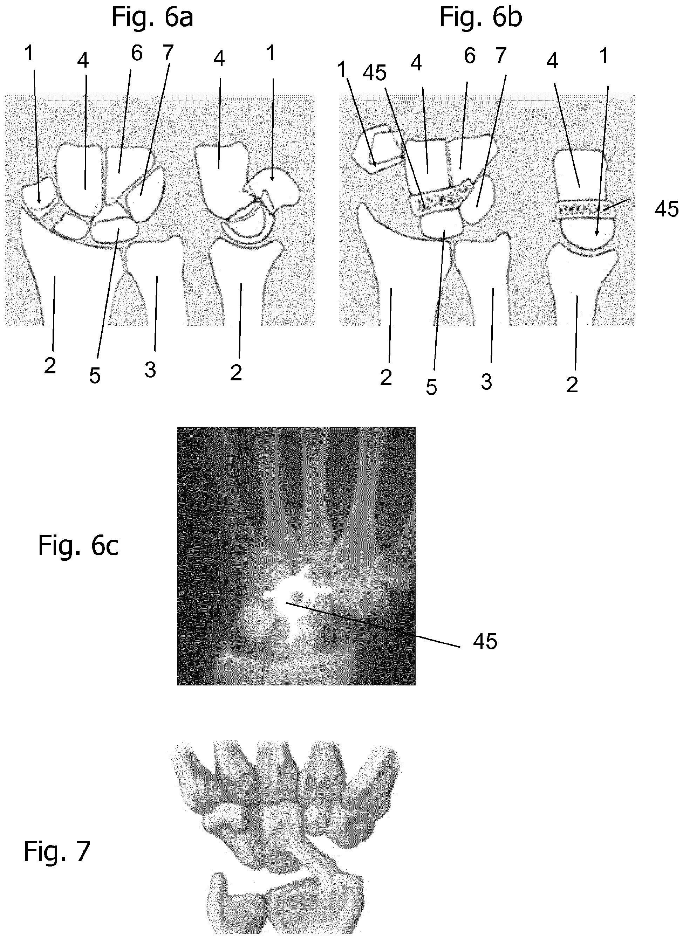

If these techniques do not lead to healing of the scaphoid bone, a partial fusion would be the next step as shown in Fig. 6a and 6b. For this reason, the complete scaphoid is excised and a fusion of the capitate 4, hamate 6, lunate 5 and triquetrum 7 is performed, commonly referred to as a 4-corner fusion. The fusion element 45 connects in Fig. 6b the capitate 4, lunate 5, triquetrum 7 and hamate 6. Fig. 6c shows a partially fused wrist treated by 4-corner fusion in an x-ray scan.

If the capitate head and the lunate fossa of the distal radius is still in good condition and covered by cartilage, a proximal row carpectomy (PRC) can be performed alternatively as shown in Fig. 7. This procedure is only during SNAC stage 1 and early stage 2 possible whereas a 4-corner fusion is also possible in stage 3. The surgical salvage procedure in the final stage of arthrosis, (complete radio- and midcarpal arthrosis) is the complete fusion of the wrist.

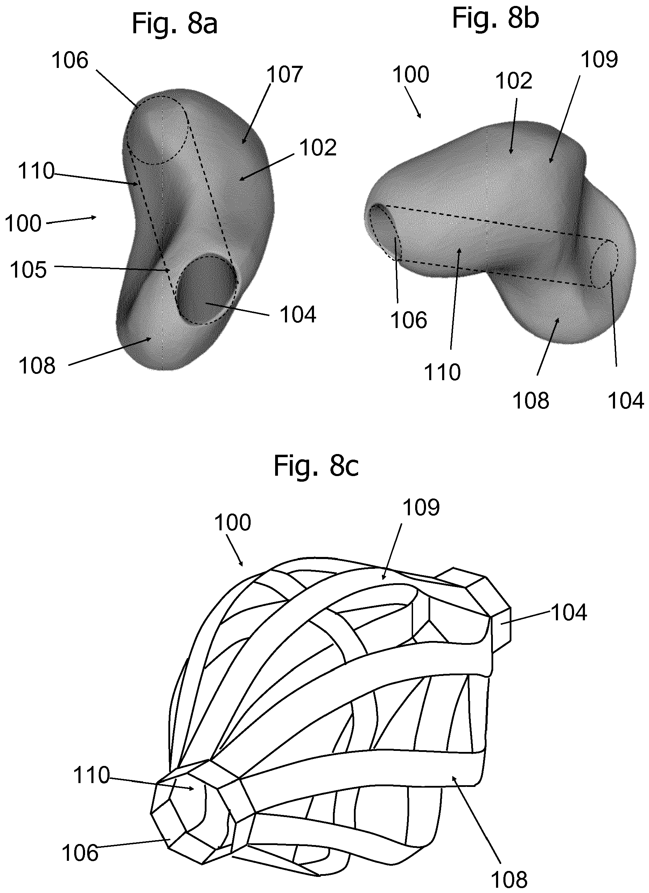

Fig. 8a shows a first view of a scaphoid prosthesis according to a first embodiment of the invention. Fig. 8b shows a second view of the scaphoid prosthesis according to Fig. 8a. The scaphoid prosthesis 100 comprises a body 102 bounded by an outer surface, whereby the outer surface is substantially corresponding to a patient's scaphoid 1. The body 102 of the scaphoid prosthesis comprises a tubular base element 105 including a first end portion 104 and a second end portion 106 and a plurality of protruding portions. By way of example, a protruding portion 107 and a protruding portion 108 are shown in Fig. 8a. A passage 110 is provided in the tubular base element for a fixation means for fixing the scaphoid prosthesis in its position.

The passage 110 is in Fig. 8a and also in Fig. 8b only partially visible, therefore, it is shown in dotted lines. The end portion 104 is configured as an opening of the passage 110 and is approximated by an ellipsoidal circumference. The shape of the circumference of the end portion 104 can deviate from the ellipsoidal structure depending on the patient's scaphoid forming the basis for the 3D model used for generating the scaphoid prosthesis.

In Fig. 8b it is also shown, that the shape of the end portion 106 may be approximated by an ellipsoidal circumference. The position of the protrusions 107, 108 in Fig. 8a and of the protrusions 108 and 109 of the Fig. 8b can be described in relation to the position of the passage 110 and the end portions 104, 106.

The tubular base element has a longitudinal axis substantially corresponding to the passage 110 in the body of the scaphoid prosthesis 100. The longitudinal axis substantially extends from the first end portion 104 to the second end portion 106. The protruding portions 107, 108, 109 have a roughly spherical or ellipsoid shape twisted about the longitudinal axis, whereby the twisting angle of the first end portion relative to the second end portion is about 90 degrees.

The scaphoid model is generated from patient data and corresponds in its shape substantially with the patient's scaphoid, thereby the scaphoid prosthesis is obtained from the scaphoid model. The scaphoid model can be designed by a computer aided design software using the anonymized patient data for calculating a shape of a replacement scaphoid of the shape of the patient's scaphoid. The shape of the replacement scaphoid can have an outer surface forming the surface of the scaphoid prosthesis, which has substantially the same shape as the surface of the patient's scaphoid. In particular, the scaphoid prosthesis can be made from a biocompatible material. The scaphoid prosthesis can be made from one of titanium, a plastic or a ceramic material. The plastic can be a biocompatible plastic. The biocompatible plastic can be made of a polymer.

By way of an example, the manufacture of a scaphoid prosthesis will be explained in the subsequent paragraph. An average size of the prosthesis was evaluated by measuring 9 scaphoids of anonymized computed tomography patient data by making use of the Geomagic Freeform® application resulting in the scaphoid prosthesis according to figures 8a/b. The scaphoid prosthesis models obtained by the Geomagic Freeform® application were segmented by making use of the Mimics® 16.0 application. The prototype was then printed in titanium. The surface finishing was performed using vibratory grinding.

According to an alternative embodiment shown in Fig. 8c, the scaphoid prosthesis is obtained from a tubular or multi-angular shape. The basis for the scaphoid prosthesis according to this embodiment can be a tubular element. This element is provided with a plurality of slits, such that a configuration is obtained, in which a plurality of striped or ribbon-shaped elements are generated. By moving the first end portion 104 towards the second end portion 106, a bulged structure is formed as the striped or ribbon-shaped elements are bent outwardly. By twisting the first bulged portion with respect the second bulged portion about the longitudinal axis, the bulged structure can be modified to correspond to the structure of a patient's scaphoid. Thus, a plurality of protrusions 108, 109 can be shaped in this manner. The thin-walled hollow structure can be reinforced by a web and/or it can be covered or coated with a biocompatible material to obtain the final shape of the scaphoid prosthesis. Advantageously, the thin-walled hollow structure is also made from a biocompatible material, in particular from a biocompatible material, which is deformable, e.g. a biocompatible metal, such as titanium.

A passage 110 can be provided in the scaphoid prosthesis for a fixation means for fixing the scaphoid prosthesis 100 in its position. The passage 110 extends from the first end portion 104 to the second end portion 106.

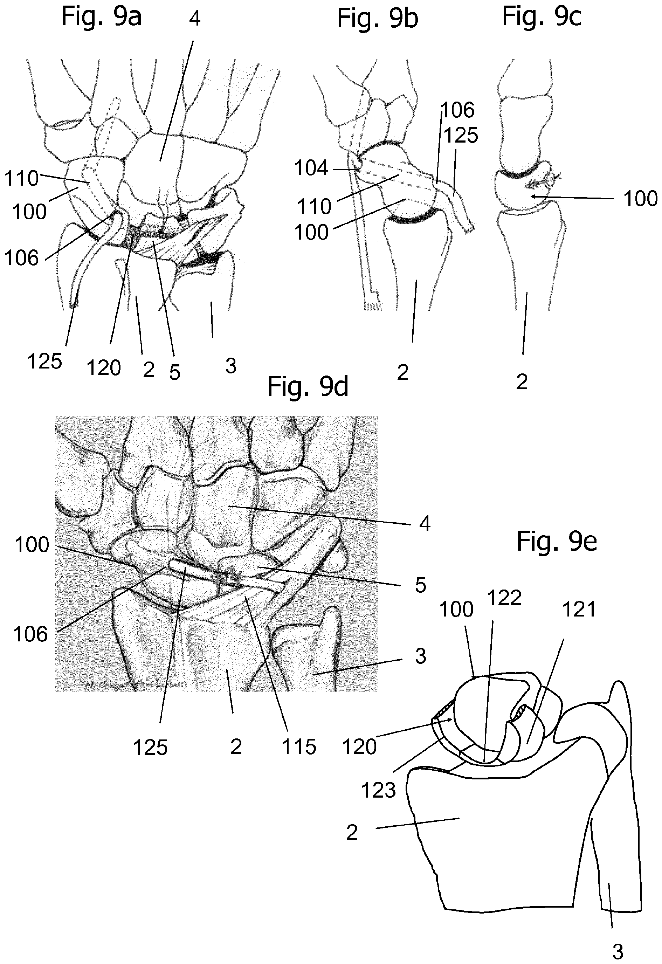

Fig. 9a, 9b, 9c show the integration of the scaphoid prosthesis 100 in the carpal bone and ligament structure. The biomechanical and functional suspension of the prosthesis derives from known procedures for scapholunate ligament 120 reconstruction, which is the ligament between the scaphoid 1 and the lunate 5 bone and one of the main stabilizers of the carpus. Other ligaments for the stabilization of the carpal bone are so called secondary stabilizers and are ligaments between the scaphoid and the carpus other than the scapholunate ligament. Fig. 9a shows the position of a scaphoid prosthesis 100 according to a configuration as described for instance in any of the preceding embodiments. The scaphoid prosthesis 100 is placed into the space of the patient's scaphoid and precisely fits into the space bounded by the radius 2, the lunate 5 and the other carpal bones. The scaphoid prosthesis is disposed with a passing 110 comprising a first end portion 104 and a second end portion 106. The first end portion 104 is shown in fig. 9b, as it is not visible in fig. 9a. A tendon strip 125 is threaded through the passage 110 and connected to itself after being passed through a dorsal radio-carpal ligament 115 as shown in Fig. 9d in more detail. Alternatively, the tendon strip 125 can only be fixed to the lunate 5 or to the lunate 5 and the triquetrum 7 without being passed through a dorsal radio-carpal ligament 115.

Fig. 9e shows a section of the scapholunate ligament 120, which is arranged on the lower circumference of the scaphoid 1 or the scaphoid prosthesis 100. The scapholunate ligament comprises a dorsal portion 121, a proximal portion 122 and a palmar portion 123.

The suspension is carried out through the passage 110 in the prosthesis using a tendon strip of the Flexor Carpi Radialis tendon (FCR) 125. A well-known technique described by Marc Garcia-Elias [8] is shown in Fig. 9d who modified several techniques for the scapholunate ligament reconstruction.

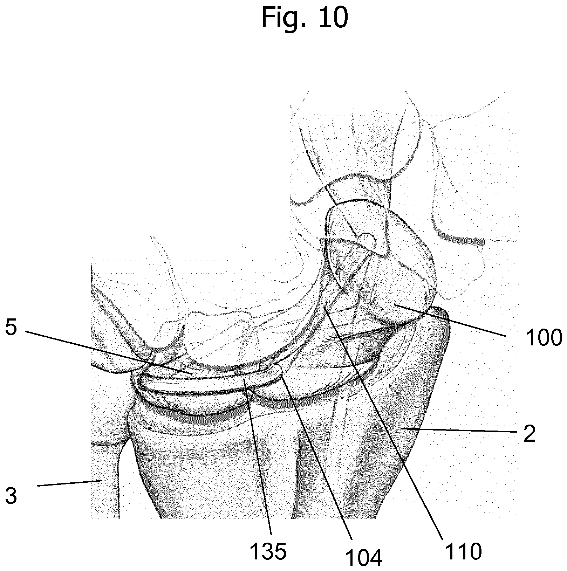

Alternatively, a minimal invasive and modified technique is described by Mark Henry or arthroscopically assisted by Fernando Corella and shown also in Fig. 10. According to this variant, the scaphoid prosthesis 100 is fixed to the lunate by directing a tendon strip of a FCR 135 around the lunate 5. The passage 110 in the scaphoid prosthesis 100 extends from the palmar surface of the scaphoid prosthesis to the dorsal surface of the scaphoid prosthesis 100. The palmar surface is located in Fig. 9f on the rear side of the drawing, the dorsal surface is visible and therefore the opening corresponding to the first end portion 104. The second opening of the second end portion 106 is located on the palmar side. The scaphoid prosthesis is shown in a transparent mode to make the passage 110 visible, which connects the first end portion 104 to the second end portion 106.

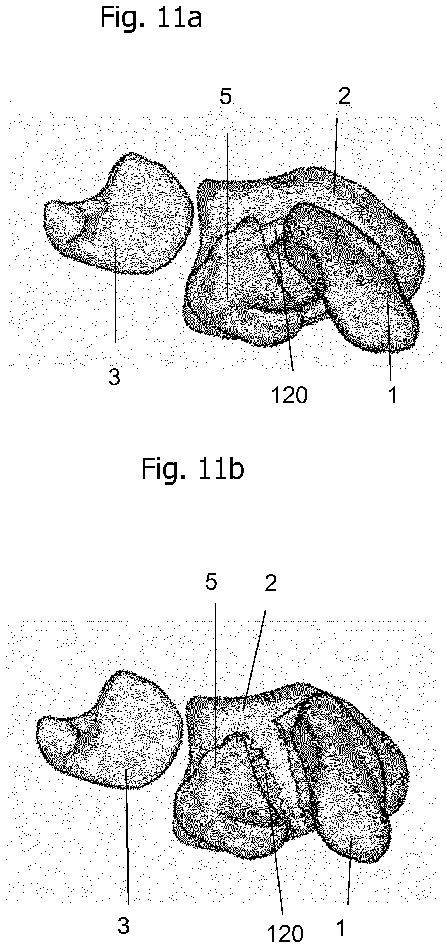

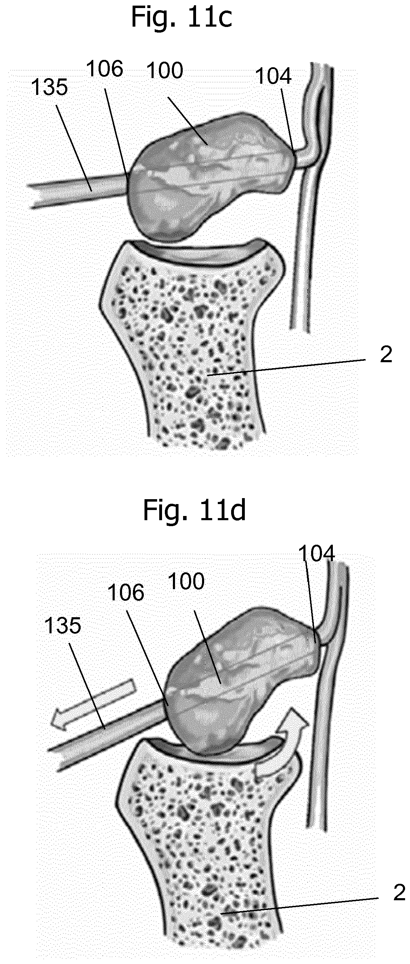

Fig. 11a-j show an alternative technique to place and fix a scaphoid prosthesis 100 to lunate 5. Fig. 11a is a top view onto the scaphoid 1 in its biomechanically correct position with respect to the lunate 5 and the radius 2 shown behind the scaphoid and the ulna 3. The scapholunate ligament 120 connecting the scaphoid and the lunate is shown in their normal position. Fig. 11b shows a rupture of the scapholunate ligament. Fig. 11c shows the first step of a scapholunate ligament reconstruction from a lateral view as proposed by Fernando Corella, with the principal modification that the patient's scaphoid 1 is substituted by the scaphoid prosthesis 100 according to any of the preceding embodiments. The scaphoid prosthesis is disposed with a passage 110. A tendon strip 135 is threaded through the passage 110 from the first end portion 104 to the second end portion 106. The tendon strip 135 can be a portion of the FCR (flexor carpi radialis) tendon or another one. In Fig. 11d it is shown, that the scaphoid prosthesis 100 is placed in the biomechanically correct position by pulling the end of the tendon strip 135 extending from the second end portion 106. Thereby the scaphoid prosthesis is rotated and/or repositioned on the joint socket of the radius 2.

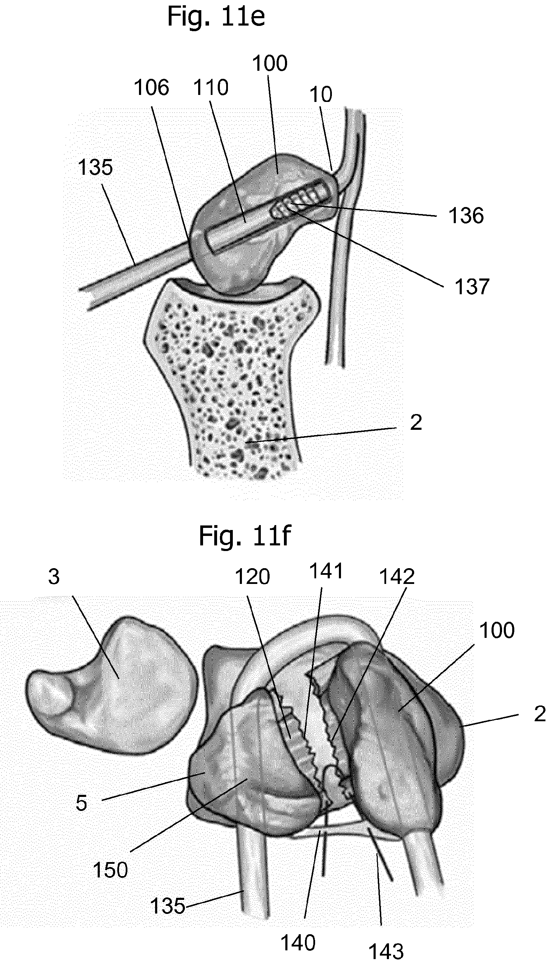

Fig. 11e shows a section of the scaphoid prosthesis 100 showing the passage 110. The tendon strip 135 extends through the passage 110. A portion of the tendon strip 135 is shown in section, which reveals the inner structure 136 of the tendon strip 135. The inner structure includes an interference screw 137, as an example of a fixation element, which is used for fixing the tendon strip 135 in its position in the passage 110. The passage 110 is at least on the location of the desired final position of the interference screw 138 is disposed with a thread corresponding to the thread of the interference screw 137. The diameter of the passage 110 may be greater than the outer diameter of the interference screw 137 in those locations, which the interference screw has to pass before reaching its final position. Alternatively, the passage may be composed of a first hole and a second hole, whereby the first hole comprises a longitudinal axis and the second hole comprises a longitudinal axis. The first and second longitudinal axes extend in a substantially parallel configuration to each other. The first hole and the second hole advantageously include a common intersection plane, such that a portion of the first hole extends into the second hole and a portion of the second hole extends into the first hole. One of the first or second holes is advantageously disposed with a thread. According to an embodiment, the surface of the passage can include a roughened portion. The roughened portion may form an anchoring portion for an interference screw. A thread may be formed in the roughened portion by the interference screw when placing the interference screw in the roughened portion. The roughened portion advantageously comprises a material which softer or at most of the same hardness as the material of the interference screw. A softer material may ease the positioning of the interference screw in the roughened portion of the passage 110.

Fig. 11f shows the fixation of the tendon strip 135 onto the lunate bone 5 on the palmar side. The lunate bone 5 is disposed with a passage 150, which receives another portion of the tendon strip 135. Thereby the scaphoid prosthesis 100 can be connected to the lunate bone 5. Fig. 11f further shows a fixation element 140, which connects the scaphoid prosthesis 100 to the lunate 5 on the palmar side. The fixation element 140 is also tied to the first and second end 141, 142 of the ruptured scapholunate ligament 120 on its dorsal side by a thread 143 and to the FCR (or other tendon) tendon, such that in particular the tendon strip 135 taken from the FCR is united by a suture to the main body of the FCR tendon. Thereby the scapholunate ligament 120 can be reconstructed and the position of the scaphoid prosthesis 100 can be stabilized.

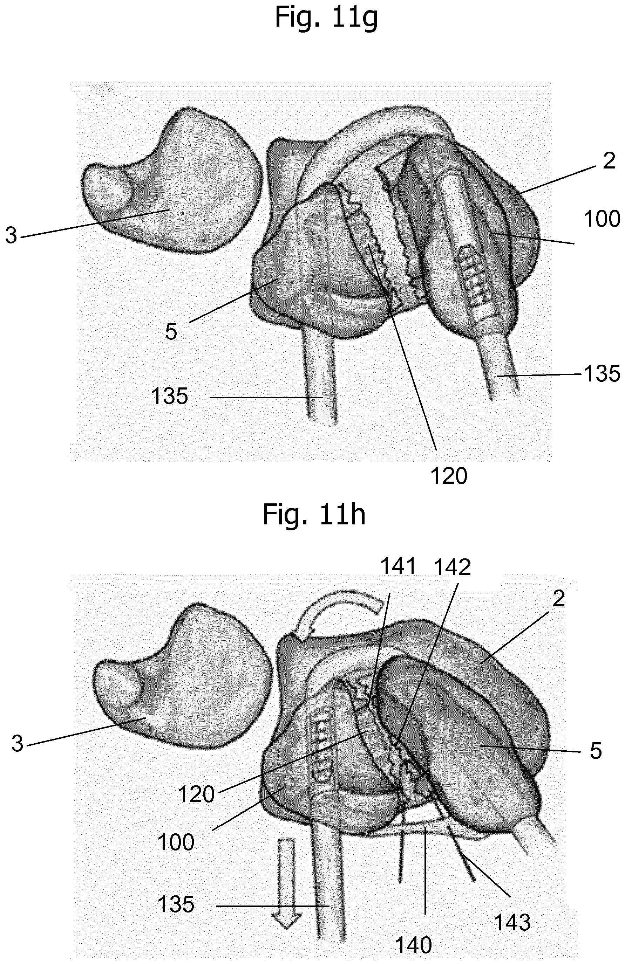

Fig. 11g shows a section of the tendon strip 135 in the scaphoid prosthesis 100.

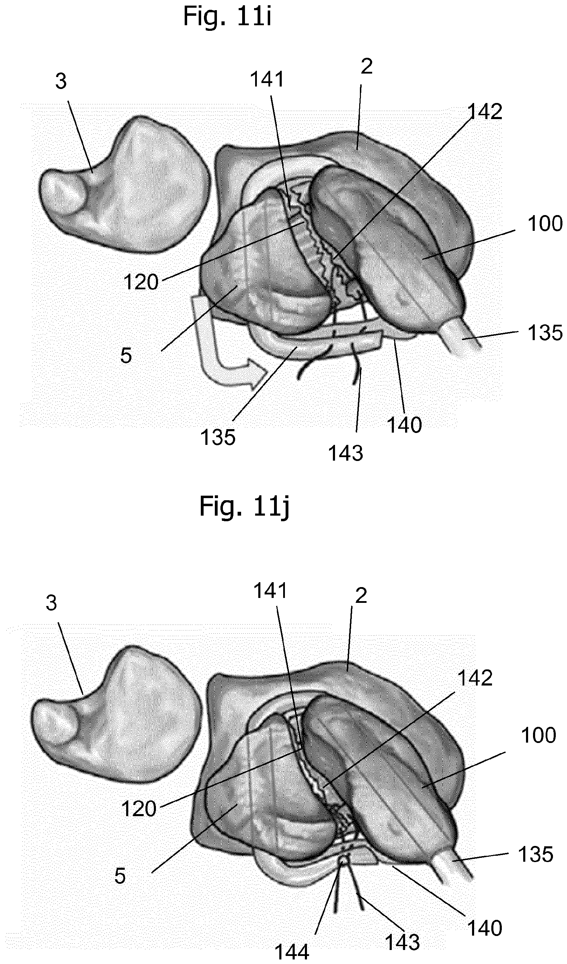

Fig. 11h shows an adjustment of the position of the scaphoid prosthesis 100 with respect to the lunate 5 by pulling the tendon strip 135. The first end 141 and the second end 142 of the ruptured scapholunate ligament 120 are moved closer to each other. In a next step shown in Fig. 11i, the end of the tendon strip 135 is attached to the fixation element 140 by passing the two ends of the thread 143 through the end of the tendon strip 135.

Fig. 11j shows that the two ends of the thread 143 are tied together to a knot 144. Thereby the tendon strip 135 is fixed in its position and the scaphoid prosthesis as well as the two ends 141, 142 of the scapholunate ligament 120 are fixed in their respective positions.

An aspect of the disclosure relates to a method for manufacturing a scaphoid prosthesis comprising a body bounded by an outer surface, wherein the outer surface is substantially corresponding to a patient's scaphoid, wherein the body of the scaphoid prosthesis comprises a tubular base element including a first end portion and a second end portion and a plurality of protruding portions wherein a single passage is provided in the tubular base element configured to engage a fixation means for fixing the scaphoid prosthesis in its position, wherein the passage is configured as a curved passage, the method comprising an additive manufacturing step.

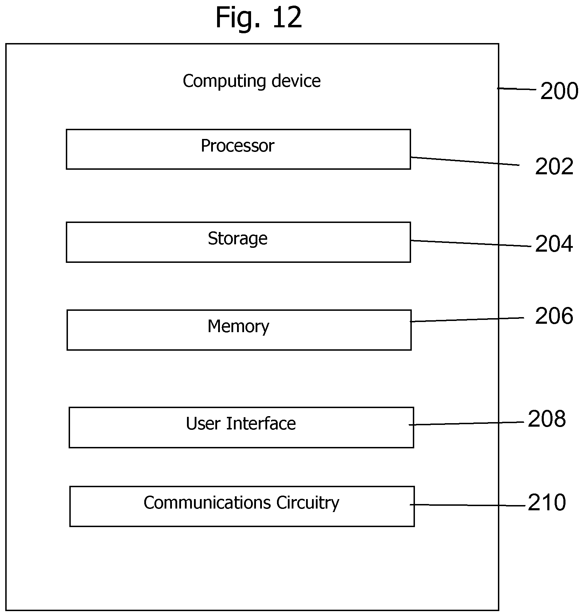

In one embodiment, the method comprising, by a computing device 200 such as shown in Fig. 12, receiving data relating to the shape of a patient's scaphoid and generating a scaphoid model, wherein the shape of the scaphoid model corresponds to the shape of the patient's scaphoid.

In one embodiment, the method comprising, by a computing device 200, generating a scaphoid model utilizing data relating to the shape of a patient's scaphoid.

A method for manufacturing a scaphoid prosthesis according to any of the preceding embodiments comprises an additive manufacturing step. In a first step of the method data are obtained relating to the shape of a patient's scaphoid, in a second step a scaphoid model can be generated by a computer aided design software. The scaphoid model can be generated from patient data. Advantageously, the scaphoid model can correspond in its shape substantially with the patient's scaphoid. In a third step of the method, the scaphoid prosthesis can be obtained from the scaphoid model by the additive manufacturing method.

A method for manufacturing a scaphoid prosthesis according to any of the preceding embodiments, wherein the scaphoid prosthesis is formed from a tubular element comprising a plurality of ribbon-shaped elements, whereby the outer shape of the scaphoid prosthesis is shaped by moving the first end portion towards the second end portion such that a roughly spherical or ellipsoidal shape is obtained, whereby the first end portion is twisted relative to the second end portion such that a scaphoid shape is obtained which corresponds roughly to the surface of the patient's scaphoid.

The computing device 200 as shown in Fig. 12, can have a processor 202 configured to generate a scaphoid model. The processor 202 can be used to run operating system applications, firmware applications, media playback applications, media editing applications, or any other application. In some embodiments, processor 202 can drive a display and process inputs received from an interface. The processor 202 can be an FPGA, ASIC, microchip, hardwired circuit, software controlled processor, DSP, or the like.

The computing device 200 can have storage 204, such as, one or more storage mediums including a hard-drive, solid state drive, flash memory, permanent memory such as ROM, any other suitable type of storage component, or any combination thereof. Storage 204 can store, for example, media data (e.g., audio or video files), application data (e.g., for implementing functions on the computing device 200), firmware, data relating to the shape of a patient's scaphoid and/or scaphoid model, and any other suitable data or any combination thereof.

The computing device 200 can have a memory 206, such as a cache memory, a semi-permanent memory, such as a RAM, and/or one or more different types of memory used for temporarily storing data. In some embodiments, the memory 206 can also be used for storing data used to operate computing device applications, or any other type of data that can be stored in the storage 204. In some embodiments, the memory 206 and the storage 204 can be combined as a single storage medium. In some embodiments, the memory 206 and the storage 204 are coupled to the processor 202.

The computing device 200 can have a user interface 208 configured to receive instructions, e.g. from a user, by way of a keyboard, keypad, touch pad, microphone, movement sensor, gesture sensor, camera, or the like. The user interface 208 can have a display/monitor of any type (LED, LCD, OLED, Plasma, CRT, or the like) and/or sound generators, such as speakers.

The computing device 200 can have communications circuitry 210, for example, any suitable communications circuitry 210 configured to connect to a communications network and to transmit communications (e.g., voice or data) from computing device 200 to other electronic devices. The communication circuitry 210 can have receivers and/or transmitters. The receivers can be configured to receive instructions from a device and thus allows a user to enter instructions into the computing device 200. The transmitters can be configured to transmit instructions from the computing device 200 and thus allow a user to send instructions from the computing device 200 to another device, such as a device used in an additive manufacturing method. The receivers and/or transmitters, and the computing device 200 corresponding thereto, can be configured to communicate over a wired connection or over a wireless connection, such as via Ethernet, LAN, WAN, Bluetooth, WiFi, IR communication, or a cloud environment. It should be apparent to those skilled in the art that many more modifications besides those already described are possible without departing from the inventive concepts herein. The inventive subject matter, therefore, is not to be restricted except in the scope of the appended claims. Moreover, in interpreting both the specification and the claims, all terms should be interpreted in the broadest possible manner consistent with the context. In particular, the terms "comprises" and "comprising" should be interpreted as referring to elements, components, or steps in a non-exclusive manner, indicating that the referenced elements, components, or steps may be present, or utilized, or combined with other elements, components, or steps that are not expressly referenced. Where the specification claims refers to at least one of an element or compound selected from the group consisting of A, B, C .... and N, the text should be interpreted as requiring only one element from the group, not A plus N, or B plus N, etc.

### References

1. AGNER O (1963) TREATMENT OF NON-UNITED NAVICULAR FRACTURES BY TOTAL EXCISION OF THE BONE AND THE INSERTION OF ACRYLIC PROSTHESES. Acta Orthop Scand 33:235-245.
2. Spingardi O, Rossello MI (2011) The total scaphoid titanium arthroplasty: A 15-year experience. Hand (N Y) 6:179-184. doi: 10.1007/s11552-010-9315-3
3. Henry M (2013) Reconstruction of Both Volar and Dorsal Limbs of the Scapholunate Interosseous Ligament. YJHSU 38:1625-1634. doi: 10.1016/j.jhsa.2013.05.026
4. Corella Fernando (2013), Del Cerro MD M, MD MO, PhD RL-G (2013) Arthroscopic Ligamentoplasty of the Dorsal and Volar Portions of the Scapholunate Ligament. YJHSU 38:2466-2477. doi: 10.1016/j.jhsa.2013.09.021
5. Zaidemberg C, Siebert JW, Angrigiani C (1991) A new vascularized bone graft for scaphoid nonunion. YJHSU 16:474-478.
6. Mathoulin C, Haerle M (1998) Vascularized bone graft from the palmar carpal artery for treatment of scaphoid nonunion. J Hand Surg Br 23:318-323.
7. Bürger HK, Windhofer C, Gaggl AJ, Higgins JP (2013) Vascularized Medial Femoral Trochlea Osteocartilaginous Flap Reconstruction of Proximal Pole Scaphoid Nonunions. YJHSU 38:690-700. doi: 10.1016/j.jhsa.2013.01.036
8. Garcia-Elias M, Lluch AL, Stanley JK (2006) Three-ligament tenodesis for the treatment of scapholunate dissociation: indications and surgical technique. YJHSU 31:125-134. doi: 10.1016/j.jhsa.2005.10.011

## Claims

1. A scaphoid prosthesis (100) comprising a body (102) bounded by an outer surface (101), wherein the outer surface (101) corresponds to the surface of the patient's scaphoid (1), wherein the body (102) of the scaphoid prosthesis comprises a tubular base element (105) including a first end portion (104) and a second end portion (106) and a plurality of protruding portions (107, 108, 109) wherein a single passage (110) is provided in the tubular base element (105) configured to engage a fixation means for fixing the scaphoid prosthesis (100) in its position, **characterized in that** the passage is configured as a curved passage.

2. The scaphoid prosthesis (100) of claim 1, wherein the passage (110) is positioned in the body (102) in such a way that the distance between a passage wall and the body surface measured along any line intersecting with a longitudinal axis is substantially uniform in any cross-sectional area arranged normally to the longitudinal axis of the passage (110).

3. The scaphoid prosthesis (100) of claim 2, wherein the passage (110) extends from the first end portion (104) to the second end portion (106).

4. The scaphoid prosthesis (100) according to any one of the preceding claims, wherein the passage (110) comprises an attachment portion.

5. The scaphoid prosthesis (100) according to any one of the preceding claims, wherein the passage (110) comprises first hole and a second hole, wherein the first hole comprises a first longitudinal axis and the second hole comprises a second longitudinal axis, wherein the first and second longitudinal axes are arranged in an angle with respect to each other.

6. The scaphoid prosthesis (100) according to any one of the preceding claims, the body comprising a contralateral side, wherein the body (102) is anatomically contoured based on data from a database.

7. The scaphoid prosthesis (100) according to any one of the preceding claims, wherein the protruding portions (107, 108, 109) have a substantially spherical or ellipsoid shape twisted about a longitudinal axis, wherein the twisting angle of the first end portion (104) relative to the second end portion (106) is about 90 degrees.

8. The scaphoid prosthesis (100) according to any one of the preceding claims, whereby the scaphoid prosthesis is created utilizing a scaphoid model, wherein the scaphoid model is generated utilizing patient scaphoid data and wherein the shape of the scaphoid model represents the shape of the scaphoid prosthesis.

9. The scaphoid prosthesis (100) according to any one of the preceding claims, wherein the scaphoid prosthesis is made from a biocompatible material.

10. The scaphoid prosthesis (100) per any one of the preceding claims, wherein the scaphoid prosthesis is made from a material which comprises at least one element from the group consisting of titanium, a biocompatible plastic, and a ceramic material, wherein the biocompatible plastic comprises in particular a polymer, for instance a polyetheretherketone, or the ceramic material comprises for instance a ceramic material containing zirconia.

11. The scaphoid prosthesis (100) according to any one of the preceding claims, wherein the scaphoid prosthesis comprises an attachment portion configured to engage a fixation element, wherein the fixation element is configured to fix the scaphoid prosthesis in its position, wherein the fixation element can be configured as a tendon strip (135).

12. The scaphoid prosthesis (100) according to any one of the preceding claims, wherein the scaphoid prosthesis is obtained by an additive manufacturing method.

13. A method for manufacturing a scaphoid prosthesis (100) according to any one of the preceding claims, the method comprising an additive manufacturing step.

14. The method according to claim 13, wherein in a first step, data are obtained relating to the shape of a patient's scaphoid (1), in a second step a scaphoid model is generated by a computer aided design software, wherein the scaphoid model is generated from patient data and corresponds in its shape substantially with the patient's scaphoid, whereby the scaphoid prosthesis (100) is obtained from the scaphoid model in a third step by the additive manufacturing method.

15. The method according to one of claims 13 or 14, wherein the scaphoid model is designed by a computer aided design software using the patient data for calculating a shape of a replacement scaphoid of the shape of the patient's scaphoid.

## Patentansprüche

1. Kahnbeinprothese (100), umfassend einen Körper (102), der von einer Außenfläche (101) begrenzt wird, wobei die Außenfläche (101) der Oberfläche des Kahnbeins (1) des Patienten entspricht, wobei der Körper (102) der Kahnbeinprothese ein röhrenförmiges Grundelement (105) umfasst, das einen ersten Endabschnitt (104) und einen zweiten Endabschnitt (106) und eine Vielzahl von vorragenden Abschnitten (107, 108, 109) umfasst, wobei ein einzelner Durchgang (110) im röhrenförmigen Grundelement (105) vorgesehen ist, der für einen Eingriff mit einem Fixierungsmittel zum Fixieren der Kahnbeinprothese (100) in ihrer Position ausgelegt ist, **dadurch gekennzeichnet, dass** der Durchgang als gekrümmter Durchgang ausgelegt ist.

2. Kahnbeinprothese (100) nach Anspruch 1, wobei der Durchgang (110) im Körper (102) so angeordnet ist, dass der Abstand zwischen einer Durchgangswand und der Körperoberfläche, gemessen entlang einer beliebigen, eine Längsachse schneidenden Linie, in einer beliebigen Querschnittsfläche, die normal zur Längsachse des Durchgangs (110) angeordnet ist, im Wesentlichen gleichmässig ist.

3. Kahnbeinprothese (100) nach Anspruch 2, wobei sich der Durchgang (110) vom ersten Endabschnitt (104) zum zweiten Endabschnitt (106) erstreckt.

4. Kahnbeinprothese (100) nach einem der vorhergehenden Ansprüche, wobei der Durchgang (110) einen Befestigungsabschnitt umfasst.

5. Kahnbeinprothese (100) nach einem der vorhergehenden Ansprüche, wobei der Durchgang (110) ein erstes Loch und ein zweites Loch umfasst, wobei das erste Loch eine erste Längsachse umfasst und das zweite Loch eine zweite Längsachse umfasst, wobei die ersten und zweiten Längsachsen in einem Winkel zueinander angeordnet sind.

6. Kahnbeinprothese (100) nach einem der vorhergehenden Ansprüche, wobei der Körper eine kontralaterale Seite umfasst, wobei der Körper (102) auf Basis von Daten aus einer Datenbank anatomisch konturiert ist.

7. Kahnbeinprothese (100) nach einem der vorhergehenden Ansprüche, wobei die vorragenden Abschnitte (107, 108 109) eine im Wesentlichen kugelförmige oder ellipsenförmige Gestalt aufweisen, die um eine Längsachse verdreht ist, wobei der Verdrehungswinkel des ersten Endabschnitts (104) bezüglich des zweiten Endabschnitts (106) ungefähr 90 Grad beträgt.

8. Kahnbeinprothese (100) nach einem der vorhergehenden Ansprüche, wobei die Kahnbeinprothese unter Verwendung eines Kahnbeinmodells erzeugt wird, wobei das Kahnbeinmodell unter Verwendung von Kahnbeindaten eines Patienten erzeugt wird und wobei die Form des Kahnbeinmodells die Form der Kahnbeinprothese repräsentiert.

9. Kahnbeinprothese (100) nach einem der vorhergehenden Ansprüche, wobei die Kahnbeinprothese aus einem biokompatiblen Material gefertigt ist.

10. Kahnbeinprothese (100) nach einem der vorhergehenden Ansprüche, wobei die Kahnbeinprothese aus einem Material gefertigt ist, das zumindest ein Element aus der Gruppe von Titan, einem biokompatiblen Kunststoff und einem Keramikmaterial bestehenden umfasst, wobei der biokompatible Kunststoff insbesondere ein Polymer umfasst, beispielsweise ein Polyetheretherketon, oder das Keramikmaterial beispielsweise ein Zirkoniumdioxid enthaltendes Keramikmaterial umfasst.

11. Kahnbeinprothese (100) nach einem der vorhergehenden Ansprüche, wobei die Kahnbeinprothese einen Befestigungsabschnitt umfasst, der für einen Eingriff mit einem Fixierungselement ausgelegt ist, wobei das Fixierungselement zur Fixierung der Kahnbeinprothese in ihrer Position ausgelegt ist, wobei das Fixierungselement als Sehnenstreifen (135) ausgelegt sein kann.

12. Kahnbeinprothese (100) nach einem der vorhergehenden Ansprüche, wobei die Kahnbeinprothese durch ein additives Fertigungsverfahren erhalten wird.

13. Verfahren zur Herstellung einer Kahnbeinprothese (100) nach einem der vorhergehenden Ansprüche, wobei das Verfahren einen additiven Fertigungsschritt umfasst.

14. Verfahren nach Anspruch 13, wobei in einem ersten Schritt Daten bezüglich der Form des Kahnbeins (1) eines Patienten eingeholt werden, in einem zweiten Schritt ein Kahnbeinmodell von einer computergestützten Design-Software erzeugt wird, wobei das Kahnbeinmodell, das aus Patientendaten erzeugt wird, in seiner Form im Wesentlichen dem Kahnbein des Patienten entspricht, wodurch die Kahnbeinprothese (100) in einem dritten Schritt aus dem Kahnbeinmodell durch das additive Fertigungsverfahren erhalten wird.

15. Verfahren nach einem der Ansprüche 13 oder 14, wobei das Kahnbeinmodell von einer computergestützten Design-Software unter Verwendung der Patientendaten zur Berechnung einer Form eines Ersatzkahnbeins mit der Form des Kahnbeins des Patienten entworfen wird.

## Revendications

1. Prothèse de scaphoïde (100) comprenant un corps (102) délimité par une surface extérieure (101), la surface extérieure (101) correspondant à la surface du scaphoïde (1) du patient, le corps (102) de la prothèse de scaphoïde comprenant un élément de base tubulaire (105) comprenant une première partie d'extrémité (104) et une seconde partie d'extrémité (106) et une pluralité de parties saillantes (107, 108, 109), un passage seul (110) étant ménagé dans l'élément de base tubulaire (105) configuré pour venir en prise avec un moyen de fixation pour fixer la prothèse de scaphoïde (100) dans sa position, **caractérisé en ce que** le passage est configuré comme un passage incurvé.

2. Prothèse de scaphoïde (100) selon la revendication 1, le passage (110) étant positionné dans le corps (102) de telle sorte que la distance entre une paroi de passage et la surface de corps mesurée le long de n'importe quelle ligne coupant un axe longitudinal dans n'importe quelle zone de section transversale agencée normalement par rapport à l'axe longitudinal du passage (110) est sensiblement uniforme.

3. Prothèse de scaphoïde (100) selon la revendication 2, le passage (110) s'étendant de la première partie d'extrémité (104) à la seconde partie d'extrémité (106).

4. Prothèse de scaphoïde (100) selon l'une quelconque des revendications précédentes, le passage (110) comprenant une partie de fixation.

5. Prothèse de scaphoïde (100) selon l'une quelconque des revendications précédentes, le passage (110) comprenant un premier trou et un second trou, le premier trou comprenant un premier axe longitudinal et le second trou comprenant un second axe longitudinal, les premier et second axes longitudinaux étant agencés selon un angle l'un par rapport à l'autre.

6. Prothèse de scaphoïde (100) selon l'une quelconque des revendications précédentes, le corps comprenant un côté controlatéral, le corps (102) étant anatomiquement conformé sur la base de données d'une base de données.

7. Prothèse de scaphoïde (100) selon l'une quelconque des revendications précédentes, les parties saillantes (107, 108, 109) ayant une forme sensiblement sphérique ou ellipsoïdale torsadée autour d'un axe longitudinal, l'angle de torsion de la première partie d'extrémité (104) par rapport à la seconde partie d'extrémité (106) étant d'environ 90 degrés.

8. Prothèse de scaphoïde (100) selon l'une quelconque des revendications précédentes, la prothèse de scaphoïde étant créée au moyen d'un modèle de scaphoïde, le modèle de scaphoïde étant généré au moyen de données de scaphoïde de patient et la forme du modèle de scaphoïde représentant la forme de la prothèse de scaphoïde.

9. Prothèse de scaphoïde (100) selon l'une quelconque des revendications précédentes, la prothèse de scaphoïde étant constituée d'un matériau biocompatible.

10. Prothèse de scaphoïde (100) selon l'une quelconque des revendications précédentes, la prothèse de scaphoïde étant constituée d'un matériau qui comprend au moins un élément du groupe constitué par le titane, un plastique biocompatible et un matériau céramique, le plastique biocompatible comprenant en particulier un polymère, par exemple une polyétheréthercétone, ou le matériau céramique comprenant par exemple un matériau céramique contenant de l'oxyde de zirconium.

11. Prothèse de scaphoïde (100) selon l'une quelconque des revendications précédentes, la prothèse de scaphoïde comprenant une partie de fixation configurée pour venir en prise avec un élément de fixation, l'élément de fixation étant configuré pour fixer la prothèse de scaphoïde dans sa position, l'élément de fixation pouvant être configuré comme une bande à tendon (135).

12. Prothèse de scaphoïde (100) selon l'une quelconque des revendications précédentes, la prothèse de scaphoïde étant obtenue par un procédé de fabrication additive.

13. Procédé de fabrication d'une prothèse de scaphoïde (100) selon l'une quelconque des revendications précédentes, le procédé comprenant une étape de fabrication additive.

14. Procédé selon la revendication 13, dans une première étape, des données étant obtenues concernant la forme du scaphoïde d'un patient (1), dans une deuxième étape, un modèle de scaphoïde étant généré par un logiciel de conception assisté par ordinateur, le modèle de scaphoïde qui est généré à partir de données de patient correspondant dans sa forme sensiblement au scaphoïde du patient, la prothèse de scaphoïde (100) étant obtenue à partir du modèle de scaphoïde dans une troisième étape par le procédé de fabrication additive.

15. Procédé selon l'une des revendications 13 et 14, le modèle de scaphoïde étant configuré par un logiciel de conception assistée par ordinateur utilisant les données de patient pour calculer une forme d'un scaphoïde de remplacement de la forme du scaphoïde du patient.
